# EUROPEAN PATENT APPLICATION

(11) **EP 4 571 016 A1**
(43) Date of publication of application: **18.06.2025**
(21) Application number: 24219664.0
(22) Date of filing: 13.12.2024
(51) Int. Cl.: E04H 7/20, C12M 1/107, E04H 7/06, E04H 7/26, A01C 3/02

(54) **CONCRETE TANK FOR FERMENTATION, METHOD OF MANUFACTURE AND USE OF SUCH CONCRETE TANK FOR FERMENTATION**

(30) Priority: 13.12.2023 BE 202306006
(71) Applicant: Bio-Dynamics nv, 8710 Wielsbeke (BE)
(72) Inventor: VAN DAMME, Thomas, 8710 Wielsbeke (BE)
(74) Representative: Brantsandpatents bv

(57) **Abstract**

The present invention relates to a concrete tank for fermentation comprising a concrete floor slab, a cylindrical concrete sidewall, and a concrete roof structure, wherein the concrete roof structure is post-tensioned using one or more metal cable loops in a part of the concrete sidewall at the same height as the concrete roof structure and/or one or more metal cable loops in the concrete roof structure, wherein each loop is slidably positioned in a sleeve, wherein each loop substantially forms a circle around a center of the concrete roof structure. The invention also relates to a method for manufacturing a concrete tank for fermentation and to the use of such a concrete tank for fermentation of biomass.

## Description

### TECHNICAL FIELD

The invention relates to a concrete tank, more specifically a concrete tank for fermentation. In a second aspect, the invention also relates to a method for manufacturing such a concrete tank. In another aspect, the invention also relates to a use of the concrete tank for fermentation of biomass.

### PRIOR ART

Concrete tanks as fermentation tanks are known from the prior art. Such a tank has a concrete floor slab, a cylindrical concrete side wall and a concrete roof structure. Biomass is added to the concrete tank. An example of a suitable biomass is manure. The biomass ferments in the tank, wherein digestate and mainly methane gas is formed. This methane gas is usually used in a cogeneration plant for generating heat and electricity or purified and pumped into the gas network.

The formation of gas creates an overpressure in the concrete tank, for example, an overpressure of 50 mbar. This overpressure must be absorbed by the cylindrical sidewall and the concrete roof structure. The cylindrical wall must also withstand the pressure of the biomass on the wall. These pressure forces are much greater than the overpressure from the formed gas, so the cylindrical wall can normally withstand the overpressure without any problem.

The concrete roof structure is preferably constructed as lightly as possible. The concrete roof structure namely rests on the concrete sidewall, so the heavier the concrete roof structure, the stronger the concrete sidewall must be. The overpressure from the gas creates tensile forces in the concrete roof structure, causing small cracks to form. Additionally, it is also common to install an agitator in the concrete tank, which is suspended from the concrete roof structure. The agitator serves to stir the biomass so that the fermentation proceeds evenly and stably. The agitator has such a weight that it again causes tensile forces in the concrete roof structure, which again results in a small amount of crack formation. The formed gas contains sulfur, which can damage the concrete of the roof structure and lead to cracks. Finally, the concrete roof structure will heat up and cool down due to environmental factors, causing the concrete to expand and contract. This is again a cause of minor crack formation. A concrete roof structure is usually made of reinforced concrete. Reinforcement meshes are installed in the concrete of the roof structure. The concrete roof structure is thus constructed strong enough not to break, but the small cracks form gas leaks, allowing formed gas to escape into the environment. To avoid gas leaks, a lining is often applied to the inside of the concrete tank to make the concrete tank gas-tight. The disadvantage of such lining is that it has a short lifespan, sometimes only ten years, requiring the lining to be repaired multiple times during the lifespan of the concrete tank. Repairs may require immediate repair in case of an acute leak, causing the concrete tank to be emptied unexpectedly.

The present invention aims to at least find a solution to some of the above-mentioned problems or disadvantages.

### SUMMARY OF THE INVENTION

In a first aspect, the present invention relates to a concrete tank according to claim 1.

The one or more loops are particularly advantageous for compressing the concrete of the concrete roof structure with great force towards the center of the concrete roof structure. When the concrete roof structure heats up or when there is overpressure from gas formed in the concrete tank, radial tensile forces normally arise in the concrete, leading to cracks. An agitator in the concrete tank is also usually suspended in or around the center of the concrete roof structure, again causing radial tensile forces. By compressing the concrete towards the center of the concrete roof structure, the tensile forces are compensated, and the concrete does not crack. As a result, the concrete roof structure remains gas-tight. This is not possible with only reinforcement meshes, as the bars of the reinforcement meshes are not radially oriented. Some of the bars of the reinforcement meshes will contribute little to absorbing the tensile forces. In addition, there must first be some stretch in the concrete before reinforcement meshes can absorb tensile forces, which means that cracks can already occur. The fact that the concrete roof structure remains gas-tight is particularly beneficial because the concrete roof structure has a very long lifespan of up to fifty years or more. Even when using a lining as an additional gas-tight barrier, a defect in the lining does not pose an immediate problem and a repair or renovation of the lining can be scheduled. It is very advantageous that the concrete roof structure has a similar weight compared to the prior art. The cylindrical sidewall does not need to be unnecessarily reinforced to support a heavier concrete roof structure.

Preferred embodiments of the concrete tank are shown in claims 2 to 9.

A particular preferred embodiment of the invention relates to a device according to claim 3.

This embodiment is particularly advantageous because the concrete top layer can move independently of the underlying concrete vaults due to the sprayed insulating foam layer. This makes it possible to compress the concrete top layer without the concrete top layer cracking. An additional advantage is that this thermally insulates the concrete roof structure, resulting in more stable conditions for the fermentation in the concrete tank. Particularly advantageous is that the insulating foam layer forms an additional gas barrier.

In a second aspect, the present invention relates to a method according to claim 10.

This method has the advantage, among others, that a concrete tank can be constructed with only a limited number of additional steps, with the concrete roof structure being compressed with great force towards its center. As a result, tensile forces in the concrete of the concrete roof structure are absorbed and do not result in cracks, keeping the concrete roof structure gas-tight. It is very advantageous that the method allows for the construction of a concrete roof structure with a weight comparable to that in the prior art. The cylindrical sidewall of the concrete tank does not need to be unnecessarily reinforced to support a heavier concrete roof structure.

Preferred embodiments of the method are described in dependent claims 11-14.

In a third aspect, the present invention relates to a use according to claim 15.

This use results in a cost-effective fermentation of biomass in a concrete tank, wherein because of the concrete roof structure of the concrete tank no or only very minimal methane gas escapes from the concrete tank. Methane gas is harmful to the climate. By avoiding cracks in the concrete roof structure, a higher gas yield from the fermentation can also be obtained.

### DESCRIPTION OF THE DRAWINGS

**Figure 1** shows a top view of a concrete tank according to an embodiment of the present invention.
**Figure 2** shows a cross-section in a vertical plane of a concrete tank according to an embodiment of the present invention.
**Figure 3** shows a detailed cross-section in a vertical plane of a concrete tank according to an embodiment of the present invention.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

The terms "comprise," "comprising," "consist of," "consisting of," "provided with," "include," "including," "contain," "containing," are synonyms and are inclusive or open terms that indicate the presence of what follows, and which do not exclude or prevent the presence of other components, characteristics, elements, members, steps, as known from or disclosed in the prior art.

Quoting numeric intervals by the endpoints includes all integers, fractions, and/or real numbers between the endpoints, including those endpoints.

In a first aspect, the invention relates to a concrete tank for fermentation.

The concrete tank comprises a concrete floor slab, a cylindrical concrete sidewall, and a concrete roof structure.

The concrete floor slab is preferably a disk-shaped floor slab. The concrete floor slab is preferably reinforced using reinforcement meshes. The reinforcement meshes have a mesh size of at most 150 mm, preferably at most 100 mm. The bars of the reinforcement meshes preferably have a diameter of at least 10 mm, more preferably at least 12 mm. Preferably, the floor slab comprises an upper reinforcement and a lower reinforcement. The upper reinforcement is preferably at a distance of at least 30 mm from a top side of the concrete floor slab, more preferably at least 40 mm. The upper reinforcement is preferably at a distance of at most 70 mm from the top side of the concrete floor slab, more preferably at most 60 mm, even more preferably at most 55 mm, and even more preferably at most 50 mm. Bars of reinforcement meshes of the upper reinforcement preferably have a diameter of at least 14 mm, more preferably at least 16 mm. The lower reinforcement is preferably at a distance of at least 30 mm from an underside of the concrete floor slab, more preferably at least 40 mm. The lower reinforcement is preferably at a distance of at most 70 mm from the underside of the concrete floor slab, more preferably at most 60 mm, even more preferably at most 55 mm, and even more preferably at most 50 mm. The concrete floor slab is placed directly on a ground surface or on a foundation. The foundation is a trench or pile foundation. Preferably, the foundation is a pile foundation.

The concrete cylindrical sidewall is preferably cast in place. The concrete cylindrical sidewall is preferably cast layer by layer. The concrete sidewall is preferably reinforced. The concrete sidewall is reinforced using reinforcement meshes. Alternatively, the concrete sidewall is reinforced with pre-tensioned or post-tensioned cables, wherein the pre-tensioned or post-tensioned cables are placed in circular loops in the concrete cylindrical sidewall. The cylindrical loops are here concentric with a circumference of the concrete cylindrical sidewall. Alternatively, the concrete cylindrical sidewall is reinforced with a combination of at least two elements from a group consisting of reinforcement meshes, pre-tensioned cables, and post-tensioned cables. Preferably, the concrete cylindrical sidewall is reinforced at least with post-tensioned cables. The concrete cylindrical sidewall is preferably insulated. This is advantageous for stable conditions in the concrete tank for fermentation. The concrete cylindrical sidewall is optionally heated using piping in the cylindrical sidewall.

The concrete roof structure is preferably reinforced. The concrete roof structure is preferably reinforced using reinforcement meshes. The reinforcement meshes preferably have a mesh size of at most 150 mm. The bars of the reinforcement meshes preferably have a diameter of at least 6 mm, more preferably at least 8 mm. Preferably, the concrete roof structure comprises an upper reinforcement and a lower reinforcement. The upper reinforcement is preferably at a distance of at least 30 mm from a top side of the concrete roof structure, more preferably at least 40 mm. The upper reinforcement is preferably at a distance of at most 70 mm from the top side of the concrete roof structure, more preferably at most 60 mm, even more preferably at most 55 mm, and even more preferably at most 50 mm. The lower reinforcement is preferably at a distance of at least 30 mm from an underside of the concrete roof structure, more preferably at least 40 mm. The lower reinforcement is preferably at a distance of at most 70 mm from the underside of the concrete roof structure, more preferably at most 60 mm, even more preferably at most 55 mm, and even more preferably at most 50 mm.

The concrete roof structure is a cast-in-place roof structure. For this, a temporary scaffolding is built inside the concrete tank to support a formwork for casting the concrete roof structure.

Alternatively, the concrete roof structure comprises concrete crossbeams. The crossbeams form a span between the cylindrical concrete sidewall. Preferably, the concrete roof structure comprises at least two crossbeams. The concrete crossbeams are supported at both ends at the level of the cylindrical sidewall. The concrete crossbeams are, for example, supported at each end by a concrete pillar. The concrete crossbeams are, for example, supported at each end by a bearing corbel. Preferably, the concrete crossbeams are supported by a bearing corbel. The bearing corbel is comprised in the cylindrical concrete sidewall. This is advantageous because it does not waste storage space in the concrete tank on concrete pillars. The concrete crossbeams are preferably parallel. Concrete vaults are placed on the concrete crossbeams. The concrete vaults are supported by at least two crossbeams or by at least one crossbeam and the cylindrical concrete sidewall. It is clear that the concrete vaults can be supported by at least two crossbeams and by the cylindrical concrete sidewall. The concrete vaults are preferably prefabricated concrete vaults. A concrete top layer is preferably cast on the concrete vaults.

Optionally, the concrete tank includes an agitator suspended from the concrete roof structure.

According to a preferred embodiment, the concrete roof structure is post-tensioned using one or more loops of metal cable in a part of the concrete sidewall at the same height as the concrete roof structure and/or one or more loops of metal cable in the concrete roof structure. By post-tensioned, it is meant that the metal cable is only tensioned after the concrete is cast and has hardened, not before the concrete is cast as with pre-tensioned cables. The metal cable is preferably a steel cable. A metal cable is part of a single loop. A loop optionally comprises multiple cables, preferably two cables, more preferably three cables.

Each loop is slidably positioned in a sleeve. It is clear that the metal cable is slidably positioned in the sleeve. The sleeve is preferably a plastic sleeve that is placed in a formwork for the concrete roof structure before casting the concrete and which is cast into the concrete. The sleeve is advantageous because it prevents the metal cable from being fixed to the concrete and allows it to be tensioned after the concrete has hardened. At a point where the loop is closed, an additional formwork is placed, into which no concrete is initially cast. At the point where the loop is closed, an anchor is placed. The anchor is suitable for post-tensioning the metal cable. Such anchors are known from the prior art. Because no concrete is initially cast at the point where the loop is closed, the anchor is accessible, and the metal cable can be tensioned after the concrete has hardened. The anchor is preferably cast on with concrete after the metal cable is post-tensioned, ensuring the anchor is firmly integrated into the concrete roof structure. This protects the anchor and the metal cable against corrosion.

Each loop substantially forms a circle around a center of the concrete roof structure. This means that a loop is circular but does not necessarily form a perfect circle, for example, because the loop deforms locally during the casting of the concrete or because the loop is not laid perfectly in a circle before the concrete is cast. Preferably, the loop lies close to an imaginary circle, wherein a center of the circle is the center of the concrete roof structure, and each point of the loop is at a distance of at most 50 cm from the imaginary circle, wherein the distance is measured along a radius of the circle. Preferably, the mentioned distance is at most 40 cm, more preferably at most 30 cm, even more preferably at most 20 cm, and even more preferably at most 10 cm.

The one or more loops are particularly advantageous for compressing the concrete of the concrete roof structure with great force towards the center of the concrete roof structure. In the case of loops of metal cable in the cylindrical concrete sidewall, the concrete of the concrete roof structure is compressed because the cylindrical concrete sidewall is pressed against the concrete roof structure at the level of the concrete roof structure. In the case of loops of metal cable in the concrete roof structure, the concrete of the concrete roof structure is directly compressed. Preferably, the concrete tank comprises at least loops of metal cable in the concrete roof structure.

When the concrete roof structure heats up or when there is overpressure from gas formed in the concrete tank, radial tensile forces normally arise in the concrete, leading to cracks. An agitator in the concrete tank is also usually suspended in or around the center of the concrete roof structure, again causing radial tensile forces. By compressing the concrete towards the center of the concrete roof structure, the tensile forces are compensated, and the concrete does not crack. As a result, the concrete roof structure remains gas-tight. This is not possible with only reinforcement meshes, as the bars of the reinforcement meshes are not radially oriented. Some of the bars of the reinforcement meshes will contribute little to absorbing the tensile forces. In addition, there must first be some stretch in the concrete before reinforcement meshes can absorb tensile forces, which means that cracks can already occur. The fact that the concrete roof structure remains gas-tight is particularly beneficial because the concrete roof structure has a very long lifespan of up to fifty years or more. Even when using a lining as an additional gas-tight barrier, a defect in the lining does not pose an immediate problem and a repair or renovation of the lining can be scheduled. It is very advantageous that the concrete roof structure has a similar weight compared to the prior art. The cylindrical sidewall does not need to be unnecessarily reinforced to support a heavier concrete roof structure.

According to a preferred embodiment, the concrete roof structure is post-tensioned with multiple metal cables in the concrete roof structure. The metal cable is preferably a steel cable. Each metal cable is slidably positioned in a sleeve. Multiple metal cables can be slidably positioned in a single sleeve. The sleeve is as described in a previously discussed embodiment. Each metal cable forms a radius from a center of the concrete roof structure. A radius is formed by one or more metal cables. The concrete roof structure comprises multiple radii formed by one or more cables. Preferably, the radii are evenly distributed around the center of the concrete roof structure. Preferably, there is an angle of at most 20° between two radii, more preferably at most 15°, even more preferably at most 10°, and even more preferably at most 5°. Each metal cable is attached at a first end to a fixed point in the concrete roof structure. At a point of a second opposite end of the metal cable, an additional formwork is placed, into which no concrete is initially cast. At the point of the second end of the metal cable, an anchor is placed. The anchor is suitable for post-tensioning the metal cable. Because no concrete is initially cast at the point of the second end of the metal cable, the anchor is accessible, and the metal cable can be tensioned after the concrete has hardened. The anchor is preferably cast on with concrete after post-tensioning the metal cable as described above, ensuring the anchor is firmly integrated into the concrete roof structure.

The radii formed by metal cable are particularly advantageous for compressing the concrete of the concrete roof structure with great force towards the center of the concrete roof structure. This provides similar advantages as for a previously described embodiment wherein the cylindrical concrete sidewall or the concrete roof structure comprises multiple loops of metal cable. The loops have the additional advantage over the radii that the compression of the concrete roof structure is very uniform, whereas with the radii formed by metal cable, there may be reduced compression between the radii.

According to a preferred embodiment, the concrete roof structure comprises concrete crossbeams, wherein the concrete crossbeams form a span between the cylindrical sidewall, wherein concrete vaults are placed on the concrete crossbeams, and a concrete top layer is cast on the concrete vaults. This corresponds to a previously described embodiment of the concrete roof structure. The one or more post-tensioned loops or radii are placed in the concrete top layer. The crossbeams and the concrete vaults can be cast in a factory or in situ at the concrete tank. When casting the concrete crossbeams and the concrete vaults in situ, they can be cast at ground level. The top layer is preferably at least 10 cm thick, more preferably at least 12 cm, even more preferably at least 14 cm, and even more preferably at least 15 cm.

This embodiment is advantageous because the crossbeams and the concrete vaults can be pre-cast and then placed in position in the concrete tank. No scaffolding is needed in the concrete tank to cast the concrete roof structure, which simplifies the construction of the concrete tank. The concrete top layer is cast in place on the concrete vaults and is a monolithic structure. This means that the concrete of the concrete top layer is cast in one go or is cast in a structurally similar manner. Because the one or more loops of metal cable or radii are placed in the concrete top layer, the same advantages are obtained as with a concrete roof structure cast in situ.

According to a further embodiment, an insulating foam layer is sprayed between the concrete vaults and the concrete top layer. A non-limiting example of a suitable insulating foam layer is a polyurethane insulating foam layer.

This embodiment is particularly advantageous because the concrete top layer can move independently of the underlying concrete vaults due to the sprayed insulating foam layer. The insulating foam layer allows for limited movement. This makes it possible to compress the concrete top layer without the concrete top layer cracking. An additional advantage is that this thermally insulates the concrete roof structure, resulting in more stable conditions for the fermentation in the concrete tank. Particularly advantageous is that the sprayed insulating foam layer forms an additional gas barrier, as the sprayed insulating foam layer forms a continuous gas-tight layer. So even if there is a crack in the concrete top layer, the chance of a leak is minimal because this crack is very likely closed by the sprayed insulating foam layer.

According to a preferred embodiment, a foil is placed between the concrete vaults and the concrete top layer. The foil is preferably gas-tight. This is advantageous as an additional gas-tight barrier. The foil is additionally advantageous for decoupling the concrete top layer and the concrete vaults. The foil is preferably a polyethylene foil, a polypropylene foil, or a foil made of another suitable material.

This embodiment can be advantageously combined with a previously described embodiment where an insulating foam layer is sprayed between the concrete vaults and the concrete top layer. The foil and the insulating foam layer enhance each other's decoupling effect and both provide an additional gas-tight barrier. The foil is placed between the concrete vaults and the insulating foam layer or between the insulating foam layer and the concrete top layer. Preferably, the foil is placed between the concrete vaults and the insulating foam layer.

According to a preferred embodiment, the concrete crossbeams and the concrete vaults are placed within the cylindrical concrete sidewall. The concrete top layer lies on top of the cylindrical concrete sidewall. This is advantageous because when the concrete top layer is compressed by the one or more loops of metal cable or by the radii, the concrete top layer still rests on the cylindrical concrete sidewall. In a radial direction towards the center of the concrete roof structure, no gap is formed between the cylindrical concrete sidewall and the concrete top layer, which is advantageous to avoid gas leaks.

According to a further embodiment, the cylindrical concrete sidewall includes an upright joint sealing strip at an upper side. Preferably the joint sealing strip is a joint sealing strip made of polyvinyl chloride. The joint sealing strip is cast into the cylindrical concrete sidewall. The joint sealing strip is also cast into the concrete top layer. The joint sealing strip is advantageous for a gas-tight connection of the concrete top layer to the cylindrical concrete sidewall. Preferably, a bituminous bearing material is applied on both sides of the joint sealing strip on top of the cylindrical concrete sidewall. This is also described as bitumen or roofing felt. This is advantageous for additional sealing between the cylindrical concrete sidewall and the concrete top layer.

According to a preferred embodiment, joints between the concrete vaults are filled with mortar. The joints are made gas-tight at the bottom using an elastic joint sealant. A non-limiting example of a suitable elastic joint sealant is, for example, a polyurethane resin, such as Sikafloor^{®}-406. This is advantageous for making the many joints between the concrete vaults as gas-tight as possible, so that the concrete vaults also serve as a gas barrier. Preferably, channels in the concrete vaults are also filled with concrete grout at the ends of the concrete vaults, so that the channels do not form a path for gas formed in the concrete tank.

According to a preferred embodiment, a rubber coating is applied to the concrete vaults. The rubber coating is applied to the top side of the concrete vaults. The rubber coating forms an additional barrier for formed gas. The rubber coating is particularly advantageous in combination with an insulating foam layer and/or a foil between the concrete vaults and the concrete top layer. The rubber coating is also advantageous for protecting the concrete vaults from damage, for example, by sulfur fumes that may be present in the formed gas.

This embodiment can be advantageously combined with a previously described embodiment where an insulating foam layer is sprayed between the concrete vaults and the concrete top layer.

According to a preferred embodiment, the concrete crossbeams are laid at both ends on a bearing corbel comprised in the concrete sidewall. Each bearing corbel includes at least one upright threaded rod. The threaded rod is preferably a metal threaded rod, more preferably a steel threaded rod. The threaded rod preferably has a diameter of at least 20 mm, more preferably at least 22 mm, even more preferably at least 24 mm, and even more preferably at least 25 mm.

The at least one upright threaded rod is incorporated at one end of a concrete support beam in an opening in the concrete support beam. The opening is preferably an upright opening right through the concrete support beam. The upright threaded rod preferably extends over at least 85% of the length of the opening, more preferably at least 90%, and even more preferably at least 95%. Openings at a first end of the concrete support beam are poured. The openings at the first end are preferably poured with concrete grout. Openings at a second opposite end of the concrete support beam are not poured and made gas-tight using an elastic joint sealant.

This embodiment is advantageous because the concrete support beams are firmly anchored to the bearing corbel using the at least one threaded rod, while the openings for the at least one threaded rod do not form a passage for formed gas. By using an elastic joint sealant at the second end, the concrete support beams can settle while the openings remain gas-tight.

According to a preferred embodiment, a protective layer is applied to the concrete interior surfaces of the tank to protect the concrete and make the concrete tank gas-tight. Biomass and the formed gas contain sulfur, which affects the concrete interior surfaces and reduces the lifespan of the concrete tank. The protective layer forms an additional gas barrier. Non-limiting examples of possible protective layers include a rubber coating or rubber layer, an epoxy coating, an acrylate coating, and a plastic lining in the form of a foil. The plastic lining in the form of a foil is preferably at least 1 mm thick. The plastic lining in the form of a foil is preferably at most 4 mm thick. A non-limiting list of suitable materials for the plastic lining includes polyethylene, polyvinyl chloride, polypropylene. The plastic lining preferably includes a flat side and a side with hooks. The side with hooks is particularly advantageous for good adhesion of the plastic lining in concrete when concrete is cast in situ. For example, the plastic lining is placed in formwork for the concrete sidewall when the concrete sidewall is cast in situ. Preferably, a plastic lining is applied as a protective layer on the concrete sidewall and the floor slab. A plastic lining is more resistant to abrasive action by biomass than, for example, a rubber layer or a coating.

In combination with a previously described embodiment where the concrete roof structure includes concrete crossbeams on which concrete vaults are placed, the protective layer is applied at least on the concrete crossbeams, on the concrete vaults, on any bearing corbels included in the cylindrical concrete sidewall, and in rebates. In this context, a rebate refers to a space between an end of a concrete crossbeam and the cylindrical concrete sidewall and, if a bearing corbel is present, between an underside of a concrete crossbeam and the bearing corbel. Applying the protective layer in the rebate means that the protective layer is applied in this space on the cylindrical concrete sidewall, on the end of the concrete crossbeam, and, if applicable, on an underside of the concrete crossbeam and on the bearing corbel.

According to an embodiment, at least four individual loops of metal cables are post-tensioned in a single anchor. The loops can, as previously described, comprise one or several metal cables. The loops are placed in a cross shape in the anchor, as seen in a cross-section perpendicular to the metal cables. At least two loops are ends of a standing leg of the cross shape, and two loops are ends of a lying leg of the cross shape. Multiple loops in a single anchor are advantageous for very strong local compression of the concrete of the concrete roof structure. The cross shape is very advantageous for achieving good compression of the concrete over the entire height of the concrete roof structure and, through the lying leg of the cross shape, also for achieving maximum compression centrally in the concrete roof structure in a height direction.

According to an embodiment, metal cables of each loop or radius are tensioned with a force of at least 700 kN, preferably at least 800 kN, even more preferably at least 900 kN, and even more preferably at least 1000 kN. This ensures very good compression of the concrete of the concrete roof structure.

According to an embodiment, at least one loop of metal cable is placed at a distance of at most 1.00 m from an inner circumference of the concrete tank, wherein the mentioned distance is measured perpendicular to the cylindrical concrete sidewall between the cylindrical concrete sidewall and a perpendicular projection of the at least one loop on the floor slab. The mentioned distance is preferably at most 0.75 m, more preferably at most 0.50 m, and even more preferably at most 0.30 m. This embodiment is advantageous because the concrete of the concrete roof structure is compressed over a maximum surface area. Preferably, each loop of metal is at least 0.20 m from the inner circumference of the concrete tank. This ensures that there is sufficient concrete on an outer circumference of the concrete roof structure so that after the post-tensioning of the loops the concrete roof structure does not break at the outer circumference.

According to an embodiment, the concrete roof structure comprises multiple anchors. The anchors are placed on concentric circles. The distance between the concentric circles is at most 1.00 m, preferably at most 0.75 m, and more preferably at most 0.50 m. The distance is measured along a radius of the concentric circles. The concentric circles have a radius that is at least 80% of a radius of an inner circumference of the concrete tank. This embodiment is advantageous for increased compression of the concrete of the concrete roof structure using multiple anchors. By having a distance between the concentric circles of at most 1.0 m and a radius of the concentric circles that is at least 80% of the radius of the inner circumference of the concrete tank, the loops of metal cable are still as close as possible to the cylindrical concrete sidewall, compressing the concrete of the concrete roof structure over a maximum area. This embodiment is particularly advantageous in combination with a previously described embodiment where at least four loops of metal cable are post-tensioned in an anchor.

According to an embodiment, the concrete roof structure comprises an upper reinforcement and a lower reinforcement. The upper reinforcement and the lower reinforcement are as previously described. The reinforcement meshes of the upper reinforcement and the lower reinforcement are advantageous for supporting the dead weight of the concrete of the concrete roof structure and the optional agitator. In an anchor, as in a previously described embodiment, at least four loops of metal cable are post-tensioned. At least one loop lies above the upper reinforcement. The loops of the lying leg of the cross shape lie between the upper reinforcement and the lower reinforcement. The loops lie above the lower reinforcement. By placing at least one loop above the upper reinforcement, the concrete of the concrete roof structure is sufficiently compressed on an upper side to avoid cracks during expansion due to gas formation. By placing the loops of the lying leg of the cross shape between the upper reinforcement and the lower reinforcement, maximum compression is achieved centrally in the concrete roof structure in a height direction.

In a second aspect, the invention relates to a method for manufacturing a concrete tank for fermentation.

The method comprises the steps of:
- casting a concrete floor slab,
- casting a cylindrical concrete sidewall,
- casting a concrete roof structure.

The concrete floor slab is preferably cast in place. The concrete floor slab is preferably reinforced using reinforcement meshes. Preferably, the floor slab comprises an upper reinforcement and a lower reinforcement. The concrete floor slab is cast directly onto a ground surface or onto a foundation. The foundation is a trench or pile foundation.

The concrete cylindrical sidewall is preferably cast in place. The concrete cylindrical sidewall is preferably cast layer by layer. The concrete sidewall is preferably reinforced. The concrete sidewall is reinforced using reinforcement meshes. Alternatively, the concrete sidewall is reinforced with pre-tensioned or post-tensioned cables, wherein the pre-tensioned or post-tensioned cables are placed in circular loops in the concrete cylindrical sidewall. Alternatively, the concrete cylindrical sidewall is reinforced with a combination of at least two elements from a group consisting of reinforcement meshes, pre-tensioned cables, and post-tensioned cables. The concrete cylindrical sidewall is preferably insulated.

The concrete roof structure is preferably reinforced.

The concrete roof structure is a cast-in-place roof structure. For this, a temporary scaffolding is built inside the concrete tank to support a formwork for casting the concrete roof structure.

Alternatively, the concrete roof structure is formed by placing concrete crossbeams. The crossbeams form a span between the cylindrical concrete sidewall. Preferably, the concrete roof structure comprises at least two crossbeams. Concrete vaults are placed on the concrete crossbeams. The concrete vaults are supported by at least two crossbeams or by at least one crossbeam and the cylindrical concrete sidewall. It is clear that the concrete vaults can be supported by at least two crossbeams and by the cylindrical concrete sidewall. The concrete vaults are preferably prefabricated concrete vaults. A concrete top layer is preferably cast on the concrete vaults.

According to a preferred embodiment, the method includes the additional steps of placing one or more loops of metal cable in a part of the cylindrical concrete sidewall at the same height as the concrete roof structure before casting the cylindrical concrete sidewall and/or one or more loops of metal cable in the concrete roof structure before casting the concrete roof structure and post-tensioning the one or more loops after the concrete has hardened.

Each loop is slidably positioned in a sleeve. It is clear that the metal cable is slidably positioned in the sleeve. The sleeve is preferably a plastic sleeve that is placed in a formwork for the concrete roof structure before casting the concrete and which is cast into the concrete.

Each loop substantially forms a circle around a center of the concrete roof structure. We refer to a previous description of a circle substantially around a center of the concrete roof structure. At a point where the loop is closed, an additional formwork is placed, into which no concrete is initially cast. At the point where the loop is closed, an anchor is placed. The anchor is suitable for post-tensioning the metal cable. The anchor is preferably cast on with concrete after the metal cable is post-tensioned, ensuring the anchor is firmly integrated into the concrete roof structure.

This method has the advantage, among others, that a concrete tank can be constructed with only a limited number of additional steps, with the concrete roof structure being compressed with great force towards its center. As a result, tensile forces in the concrete of the concrete roof structure are absorbed and do not result in cracks, keeping the concrete roof structure gas-tight. It is very advantageous that the method allows for the construction of a concrete roof structure with a weight comparable to that in the prior art. The cylindrical sidewall of the concrete tank does not need to be unnecessarily reinforced to support a heavier concrete roof structure.

According to a preferred embodiment, the method includes the additional steps of placing multiple metal cables in the concrete roof structure before casting the concrete roof structure and post-tensioning the one or more cables after the concrete has hardened.

Each metal cable is slidably positioned in a sleeve. It is clear that multiple metal cables can be placed in a single sleeve. The sleeve is as previously described.

Each metal cable forms a radius from a center of the concrete roof structure. A radius is formed by one or more metal cables. Multiple radii are formed in the concrete roof structure. Preferably, the radii are evenly distributed around the center of the concrete roof structure. Each metal cable is attached at a first end to a fixed point in the concrete roof structure. At a point of a second opposite end of the metal cable an additional formwork is placed, into which no concrete is cast at first. At the point of the second end of the metal cable, an anchor is placed. The anchor is suitable for post-tensioning the metal cable. The anchor is preferably cast on with concrete after post-tensioning the metal cable as described above, ensuring the anchor is firmly integrated into the concrete roof structure.

This method has similar advantages to a previously described embodiment, where loops of metal cable are placed in the concrete sidewall or in the concrete roof structure. The loops have the additional advantage over the radii that the compression of the concrete roof structure is very uniform, whereas with the radii formed by metal cable, there may be reduced compression between the radii.

According to a preferred embodiment, concrete crossbeams are placed within the cylindrical concrete sidewall before casting the concrete roof structure. The concrete crossbeams form a span between the cylindrical side wall. Preferably, at least two concrete crossbeams are placed. The concrete crossbeams are supported at both ends at the level of the cylindrical sidewall. The concrete crossbeams are, for example, supported at each end by a concrete pillar. The concrete crossbeams are, for example, supported at each end by a bearing corbel. The bearing corbel is comprised in the cylindrical concrete sidewall. The concrete crossbeams are preferably placed parallel within the cylindrical concrete sidewall.

After placing the concrete crossbeams, concrete vaults are placed on the concrete crossbeams. The concrete vaults are supported by at least two crossbeams or by at least one crossbeam and the cylindrical concrete sidewall. It is clear that the concrete vaults can be supported by at least two crossbeams and by the cylindrical concrete sidewall. The concrete vaults are preferably prefabricated concrete vaults. A concrete top layer is cast on the concrete vaults. The concrete top layer is a monolithic structure. The loops or radii are placed in the concrete top layer. The loops or radii are placed before casting the concrete top layer and post-tensioned after the concrete top layer has hardened.

This embodiment is advantageous because no scaffolding is needed in the concrete tank to cast the concrete roof structure. Because the one or more loops of metal cable or radii are placed in the concrete top layer, the same advantages are obtained as with a concrete roof structure cast in situ.

According to a further embodiment, an insulating foam layer is sprayed on the concrete vaults before casting the concrete top layer. This embodiment is particularly advantageous because the concrete top layer can move independently of the underlying concrete vaults due to the sprayed insulating foam layer, allowing the concrete top layer to be compressed without cracking. Additionally advantageous is that the concrete roof structure is thermally insulated. Particularly advantageous is that the sprayed insulating foam layer forms an additional gas barrier.

According to a preferred embodiment, a foil is placed between the concrete vaults and the concrete top layer. The foil is preferably gas-tight. This is advantageous as an additional gas-tight barrier. The foil is additionally advantageous for decoupling the concrete top layer and the concrete vaults. The foil is preferably a polyethylene foil, a polypropylene foil, or a foil made of another suitable material.

According to a preferred embodiment, a protective layer is applied to the concrete interior surfaces of the concrete tank to protect the concrete and make the concrete tank gas-tight. Biomass and the formed gas contain sulfur, which affects the concrete interior surfaces and reduces the lifespan of the concrete tank. The protective layer forms an additional gas barrier. Non-limiting examples of possible protective layers include a rubber coating or rubber layer, an epoxy coating, an acrylate coating, a plastic lining in the form of a foil.

A person skilled in the art will appreciate that a method according to the second aspect is preferably carried out for manufacturing a concrete tank according to the first aspect and that a concrete tank according to the first aspect is preferably manufactured by carrying out a method according to the second aspect. Each feature described in this document, both above and below, can therefore relate to any of the three aspects of the present invention.

In a third aspect, the invention relates to a use of a concrete tank according to a first aspect for pre-acidification and/or fermentation of biomass.

This use results in advantageous pre-acidification (hydrolysis) and/or fermentation of biomass in a concrete tank, wherein because of the concrete roof structure of the concrete tank, no or only very minimal methane gas escapes from the concrete tank. Methane gas is harmful to the climate. By avoiding cracks in the concrete roof structure, a higher gas yield from the fermentation can also be obtained.

In what follows, the invention is described using non-limiting figures that illustrate the invention, and which are not intended to and should not be interpreted as limiting the scope of the invention.

### DESCRIPTION OF THE DRAWINGS

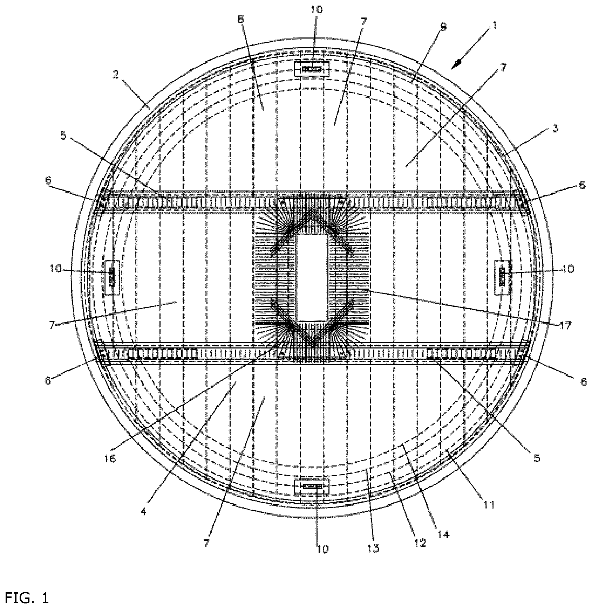

**Figure 1** shows a top view of a concrete tank according to an embodiment of the present invention.

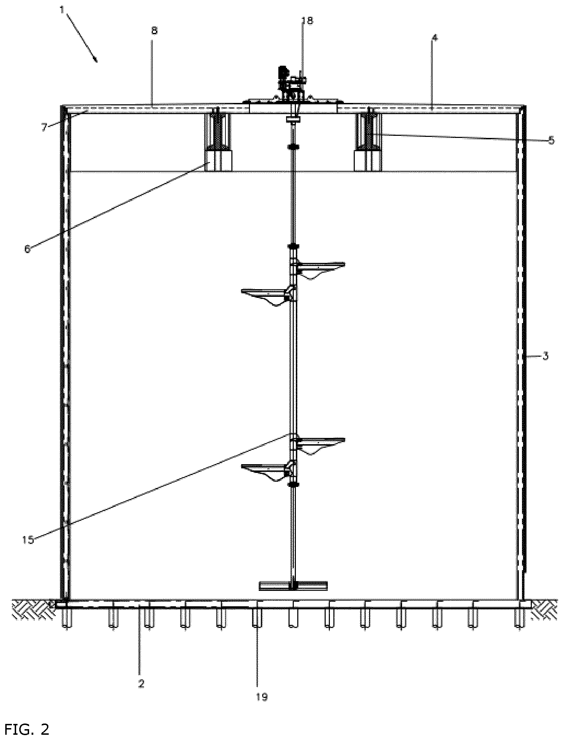

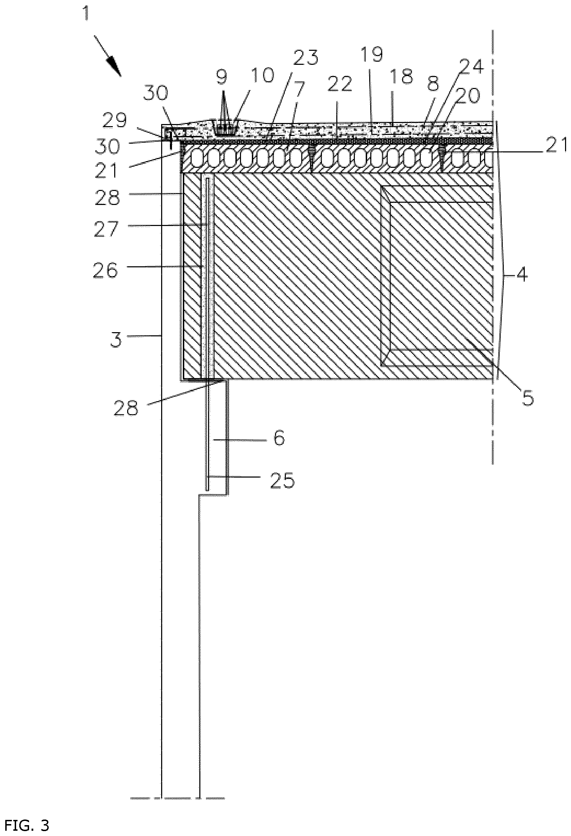

The concrete tank (1) comprises a concrete floor slab (2), a cylindrical concrete sidewall (3), and a concrete roof structure (4). Only an outer edge of the concrete floor slab (2) is visible in Figure 1. The concrete roof structure (4) includes two concrete crossbeams (5). The concrete crossbeams (5) are precast beams. The concrete crossbeams (5) form a span between the cylindrical concrete sidewall (3). Both ends of the concrete crossbeams (5) are laid on a bearing corbel (6). The bearing corbels (6) are included in the cylindrical concrete sidewall (3). Concrete vaults (7) are placed on the concrete crossbeams (5). The concrete vaults (7) are prefabricated vaults. A concrete top layer (8) is cast on the concrete vaults (7). The concrete top layer (8) conceals underlying structures of the concrete tank (1). These underlying structures are nevertheless shown in Figure 1 for illustration. The concrete roof structure (4) is post-tensioned with sixteen loops (9) of metal cable in the concrete top layer (8). The loops (9) are each individually slidably positioned in a sleeve. The loops (9) substantially form a circle around a center of the concrete roof structure (4). At a point where the loops (9) are closed, an anchor (10) is placed. The anchors (10) are suitable for post-tensioning the metal cables of the loops (9). In this embodiment, four individual loops (9) of metal cables are post-tensioned in each anchor (10). There are thus four anchors (10). Because the four loops (9) are very close together, the four loops (9) are shown as a single line at each anchor (10) in Figure 1. The four loops (9) at an anchor are more clearly visible in Figure 3. Each loop (9) in this embodiment comprises two metal cables. The four anchors (10) are placed on concentric circles (11, 12, 13, 14). Because an anchor (10) still forms a small interruption of a loop (9) and there is locally less compression of the concrete of the concrete top layer (8), the anchors (10) are placed in an orthogonal coordinate system in a horizontal plane with the center of the concrete roof structure (4) as the origin at 0°, 90°, 180°, and 270°. This ensures that the compression of the concrete of the concrete top layer (8) is as uniform as possible. In this embodiment, the concrete tank (1) has an inner circumference with a radius of 11.50 m. A first concentric circle (11) has a radius of 11.25 m, a second concentric circle (12) a radius of 10.75 m, a third concentric circle (13) a radius of 10.250 m, and a fourth concentric circle (14) a radius of 9.75 m. The concentric circles (11, 12, 13, 14) thus have a radius that is at least 80% of the radius of the inner circumference of the concrete tank (1). It will be apparent to one skilled in the art that given dimensions are illustrative and that other dimensions are possible. The concrete tank (1) in this embodiment includes an agitator (15). The agitator (15) is optional. The agitator (15) is not shown in Figure 1 but is visible in Figure 2. A concrete keystone (5) is placed on the concrete crossbeams (16). The concrete keystone (16) forms an opening in the concrete roof structure (4) through which the agitator (15) can be placed in the concrete tank (1). The concrete keystone (16) comprises an upstanding edge. The concrete top layer (8) is cast over the concrete keystone (16) up to the upstanding edge. The upstanding edge forms a tube through the concrete top layer (8). In the concrete top layer (8), additional reinforcing bars (17) are provided for locally reinforcing the concrete top layer (8) around the opening in the concrete roof structure (4).

**Figure 2** shows a cross-section in a vertical plane of a concrete tank according to an embodiment of the present invention.

The cross-section is a simplified representation of a concrete tank (1) as shown in Figure 1. The concrete floor slab (2) was cast in place. A rebar foundation (19) is placed under the concrete floor slab. Only an upper part of the rebar foundation (19) is shown in Figure 2. It is clearly visible how an agitator (15) is centrally suspended in the concrete tank (1) from the concrete roof structure (4). On top of the concrete roof structure (4), a motor (18) for driving the agitator (15) is placed. Also visible is how the concrete crossbeams (5) are placed on bearing corbels (6), included in the cylindrical concrete sidewall (3). Concrete vaults (7) are placed on these crossbeams (5). The cylindrical concrete sidewall (3) is narrower at the top, so the concrete vaults (7) also rest on the cylindrical concrete sidewall (3). The concrete top layer (8) is cast on the concrete vaults (7) and lies on top of the cylindrical concrete sidewall (3).

**Figure 3** shows a detailed cross-section in a vertical plane of a concrete tank according to an embodiment of the present invention.

Figure 3 shows in more detail a corner formed by the concrete roof structure (4) and the cylindrical concrete sidewall (3) of a concrete tank (1). The concrete tank (1) corresponds to the concrete tank (1) from Figure 1 and Figure 2. It is clearly visible how the cylindrical concrete wall (3) includes a bearing corbel (6). The bearing corbel (6) includes an upright threaded rod (25). The upright threaded rod (25) is cast into the bearing corbel (6). The upright threaded rod (25) is incorporated into an opening (26) at a first end of the concrete crossbeam (5). The opening (26) at the first end of the concrete crossbeam (5) is poured with concrete (27). At a second opposite end of the concrete crossbeam (5), the opening (26) is made gas-tight with elastic joint sealant. The second end is not shown in Figure 3. A protective layer is applied to the concrete interior surfaces of the concrete tank (1) to protect the concrete and make the concrete tank gas-tight. Between the cylindrical concrete wall (3), the concrete crossbeam (5), and the bearing corbel (6), there is a rebate (28). The protective layer is also applied to concrete surfaces in the rebate (28). The cylindrical concrete sidewall (3) includes an upright joint sealing strip (29) at an upper side. The joint sealing strip (29) is cast into the cylindrical concrete sidewall (3). The joint sealing strip (29) is also cast into the concrete top layer (8). On both sides of the joint sealing strip (29), a bituminous bearing material (30) has been applied to the top side of the cylindrical concrete sidewall (3). A rubber coating (24) is applied to the concrete vaults (7). An insulating foam layer (22) is sprayed between the concrete vaults (7) and the concrete top layer (8). In this embodiment, the insulating foam layer (22) lies on the rubber coating (24). A foil (23) is placed between the insulating foam layer (22) and the concrete top layer (8). The concrete top layer (8) includes an upper reinforcement (18) and a lower reinforcement (19) with reinforcement meshes. It is clearly visible how four loops (9) are post-tensioned in the anchor (10). The four loops (9) are placed in a cross shape in the anchor (10). Two loops (9) are ends of a lying leg of the cross shape, and two loops (9) are ends of a standing leg of the cross shape. The two loops (9) of the lying leg of the cross shape lie between the upper reinforcement (18) and the lower reinforcement (19). One loop (9) of the standing leg of the cross shape lies above the upper reinforcement (18). It is clearly visible that the concrete vaults (7) contain hollow channels (20). These channels (20) are sealed at the ends with concrete grout. Joints (21) between the concrete vaults and between a concrete vault (7) and the cylindrical concrete sidewall (3) are filled using mortar, wherein the joints (21) at the bottom have been made gas-tight using elastic joint sealant.

The numbered elements in the figures are as follows:
1. Concrete tank
2. Concrete floor slab
3. Cylindrical concrete sidewall
4. Concrete roof structure
5. Concrete crossbeam
6. Bearing corbel
7. Concrete vault
8. Concrete top layer
9. Loop
10. Anchor
11. First concentric circle
12. Second concentric circle
13. Third concentric circle
14. Fourth concentric circle
15. Agitator
16. Concrete keystone
17. Reinforcing bar
18. Upper reinforcement
19. Lower reinforcement
20. Channel
21. Joint
22. Insulating foam layer
23. Foil
24. Rubber coating
25. Threaded rod
26. Opening
27. Concrete
28. Rebate
29. Joint sealing strip
30. Bituminous bearing material

## Claims

1. Concrete tank for fermentation comprising a concrete floor slab, a cylindrical concrete sidewall, and a concrete roof structure, **characterized in that** the concrete roof structure is post-tensioned using one or more loops of metal cable in a part of the cylindrical concrete sidewall at the same height as the concrete roof structure and/or one or more loops of metal cable in the concrete roof structure, wherein each loop is slidably positioned in a sleeve, wherein each loop substantially forms a circle around a center of the concrete roof structure.

2. The concrete tank according to claim 1, **characterized in that** the concrete roof structure comprises concrete crossbeams, wherein the concrete crossbeams form a span between the cylindrical sidewall, wherein concrete vaults are placed on the concrete crossbeams and a concrete top layer is cast on the concrete vaults, wherein the one or more post-tensioned loops are placed in the concrete top layer.

3. The concrete tank according to claim 2, **characterized in that** an insulating foam layer is sprayed between the concrete vaults and the concrete top layer.

4. The concrete tank according to claim 2 or 3, **characterized in that** a foilis placed between the concrete vaults and the concrete top layer.

5. The concrete tank according to any of the preceding claims 2-4, **characterized in that** the concrete crossbeams and the concrete vaults are placed within the cylindrical concrete sidewall and that the concrete top layer lies on top of the cylindrical concrete sidewall.

6. The concrete tank according to any of the preceding claims 2-5, **characterized in that** joints between the concrete vaults are filled with mortar, wherein the joints are made gas-tight at the bottom using an elastic joint sealant.

7. The concrete tank according to any of the preceding claims 2-6, **characterized in that** a rubber coating is applied to the concrete vaults.

8. The concrete tank according to any of the preceding claims 2-7, **characterized in that** the concrete crossbeams are laid at both ends on a bearing corbel included in the concrete sidewall, wherein each bearing corbel includes at least one upright threaded rod, wherein the at least one upright threaded rod is incorporated at one end of a concrete crossbeam in an opening in the concrete crossbeam, wherein openings at a first end of the concrete crossbeam are poured with concrete and openings at a second opposite end of the concrete crossbeam are made gas-tight using an elastic joint sealant.

9. The concrete tank according to any of the preceding claims 1-8, **characterized in that** a protective layer is applied to the concrete interior surfaces of the tank to protect the concrete and make the concrete tank gas-tight.

10. Method for manufacturing a concrete tank for fermentation comprising the steps of:
- casting a concrete floor slab;
- casting a cylindrical concrete sidewall;
- casting a concrete roof structure;
**characterized in that** the method includes the additional steps of placing one or more loops of metal cable in a part of the concrete sidewall at the same height as the concrete roof structure before casting the cylindrical concrete sidewall and/or one or more loops of metal cable in the concrete roof structure before casting the concrete roof structure, wherein each loop is slidably positioned in a sleeve, wherein each loop substantially forms a circle around a center of the concrete roof structure, and post-tensioning the one or more loops after the concrete has hardened.

11. The method according to claim 10, **characterized in that** before casting the concrete roof structure, concrete crossbeams are placed within the cylindrical concrete sidewall, wherein the concrete crossbeams form a span between the cylindrical sidewall, after which concrete vaults are placed on the concrete crossbeams and a concrete top layer is cast on the concrete vaults, wherein the loops are placed in the concrete top layer.

12. The method according to claim 11, **characterized in that** an insulating foam layer is sprayed on the concrete vaults before casting the concrete top layer.

13. The method according to claim 11 or 12, **characterized in that** a foil is placed between the concrete vaults and the concrete top layer.

14. The method according to any of claims 10-13, **characterized in that** a protective layer is applied to the concrete interior surfaces of the concrete tank to protect the concrete and make the concrete tank gas-tight.

15. Use of a concrete tank according to any of claims 1-9 for pre-acidification and/or fermentation of biomass.

16. Concrete tank for fermentation comprising a concrete floor slab, a cylindrical concrete sidewall, and a concrete roof structure, **characterized in that** the concrete roof structure is post-tensioned using multiple metal cables in the concrete roof structure, wherein each metal cable is slidably positioned in a sleeve, wherein each metal cable forms a radius from a center of the concrete roof structure.
